(19) Europäisches Patentamt
European Patent Office
Office européen des brevets

(11) Veröffentlichungsnummer: **0 334 097 B1**

(12) # EUROPÄISCHE PATENTSCHRIFT

(45) Veröffentlichungstag der Patentschrift: **28.04.93**

(51) Int. Cl.$^5$: **C07C 25/18**, C07C 17/12

(21) Anmeldenummer: **89103980.2**

(22) Anmeldetag: **07.03.89**

(54) **Verfahren zur Herstellung von 4,4'-Dihalogeno-biphenylen.**

(30) Priorität: **19.03.88 DE 3809258**
**14.06.88 DE 3820192**

(43) Veröffentlichungstag der Anmeldung:
**27.09.89 Patentblatt 89/39**

(45) Bekanntmachung des Hinweises auf die Patenterteilung:
**28.04.93 Patentblatt 93/17**

(84) Benannte Vertragsstaaten:
**BE DE FR GB IT NL**

(56) Entgegenhaltungen:
**EP-A- 0 028 888**
**EP-A- 0 118 851**

**SYNTHESIS. 1985, STUTTGART DE Seiten 1157 - 1158; keith smith et al.: "Highly para-Selective Mono-Chlorination of Aromatic Compounds Under Mild Conditions by t-Butyl Hypochlorite in the Presence of Zeolites "**

**PATENT ABSTRACTS OF JAPAN vol. 11, no. 192 (C-429)(2639) 19 Juni 1987, & JP-A-62 12728**

**PATENT ABSTRACTS OF JAPAN vol. 4, no. 101 (C-19)(583) 19 Juli 1980, & JP-A-55 64532**

(73) Patentinhaber: **BAYER AG**

**W-5090 Leverkusen 1 Bayerwerk(DE)**

(72) Erfinder: **Botta, Artur, Dr.**
**Bodelschwinghstrasse 119**
**W-4150 Krefeld(DE)**
Erfinder: **Buysch, Hans-Josef, Dr.**
**Brandenburger Strasse 28**
**W-4150 Krefeld(DE)**
Erfinder: **Puppe, Lothar, Dr.**
**Am Weiher 10a**
**W-5093 Burscheid(DE)**

EP 0 334 097 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 4,4′-Dihalogeno-biphenylen durch selektive Halogenierung von Biphenylen in Gegenwart von Zeolithen als Katalysatoren, die Porenweiten von mindestens 5 Å haben.

4,4′-Dichlor- bzw. -Dibrom-biphenyl beanspruchen starkes Interesse, beispielsweise als Zwischenprodukt für die Herstellung von hochwärmestandfesten Kunststoffen, wie modifizierte Polyphenylensulfide (vgl. JP-Patentanmeldung 61/231 030 bzw. US 3.396.110 [C.A. 69 P 60 564 w]).

Die konventionelle Chlorierung von Biphenyl in Gegenwart von Lewis-Säuren führt zu einer unselektiven statistischen Substitution, wobei das 4,4′-Derivat nicht das bevorzugte ist. So liefert die Chlorierung bei 100 °C in Gegenwart von 2,5 % $FeCl_3$ eine Selektivität von nur 8 % an dem 4,4′-Dichlorisomeren; der Anteil an Trichlor-biphenyl ist mit 15 % bemerkenswert hoch, wobei bekanntlich polychlorierte Biphenyle zu den hochtoxischen Substanzklassen zählen. Nach den Angaben von US 1 946 040, US 3 226 447 und GB 1 153 746 soll der Zusatz einer Schwefelverbindung bei der Chlorierung von Benzolen die Selektivität zugunsten der para-Substitution erhöhen. Wie das Vergleichsbeispiel III weiter unten zeigt, wird aber bei der Chlorierung von Biphenyl durch Zugabe von 2,5 Gew.-% Thiophen neben 2,5 Gew.-% $FeCl_3$ die Selektivität zugunsten des 4,4′-Dichlorbiphenyls nur unwesentlich gesteigert, während polychlorierte Biphenyle mit mehr als 9 Gew.-% des Reaktionsproduktes immer noch einen beträchtlichen Anteil einnehmen.

Die Verwendung von Lewis-Säuren führt in der Praxis weiterhin bekanntlich zu erhöhten Korrosionsproblemen sowie zu einer erschwerten Aufarbeitung und Entsorgung.

Es wurde nun ein Verfahren zur Herstellung von 4,4′-Dihalogeno-biphenylen der Formel

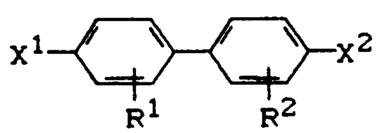

(I)

durch katalysierte Halogenierung von Biphenylen der Formel

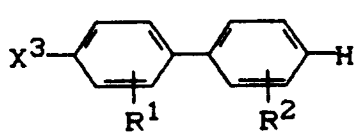

(II),

wobei in den Formeln

| | |
|---|---|
| $X^1$ und $X^2$ | unabhängig voneinander für Chlor oder Brom, bevorzugt für Chlor stehen, |
| $X^3$ | für Wasserstoff, Chlor oder Brom, bevorzugt für Wasserstoff oder Chlor, besonders bevorzugt für Wasserstoff steht und |
| $R^1$ und $R^2$ | unabhängig voneinander Wasserstoff, $C_1$-$C_4$-Alkyl (bevorzugt $C_1$-$C_2$-Alkyl, besonders bevorzugt Methyl), $C_1$-$C_4$-Alkoxy (bevorzugt $C_1$-$C_2$-Alkoxy, besonders bevorzugt Methoxy), Hydroxy, Fluor, Chlor oder Brom bedeuten, |

gefunden, das dadurch gekennzeichnet ist, daß man die Biphenyle mit Halogenierungsmitteln aus der Gruppe von $Cl_2$, $Br_2$, $SO_2Cl_2$ und $SO_2Br_2$ in Gegenwart von Zeolithen der Formel

$$M_m [mMe^1O_2 \cdot nSiO_2] \cdot q\, H_2O \qquad (III),$$

worin

| | |
|---|---|
| M | ein austauschbares Kation aus der Gruppe von Wasserstoff und den Alkalimetallen bedeutet, |
| $Me^1$ | ein dreiwertiges Element aus der Gruppe von Al, Ga, In, B und V darstellt, |
| n/m | das Verhältnis der Elemente bedeutet und Werte von 1 - 3000 annimmt und |
| q | die Menge des sorbierten Wassers bedeutet, |

wobei die Zeolithe Porenweiten von mindestens 5 Å haben, umsetzt.

Das erfindungsgemäße Verfahren erlaubt demnach den Einsatz der genannten Biphenyle, wobei eine der para-Positionen bereits durch Halogen besetzt sein kann; diese zuletzt genannte Variante erlaubt sogar die Herstellung von in 4,4′-Stellung verschieden halogenierten Biphenylen. In ganz besonders bevorzugter

Weise wird das nicht-substituierte Biphenyl umgesetzt. Am nicht-substituierten Biphenyl läßt sich die erfindungsgemäße Reaktion durch folgendes Formelschema veranschaulichen:

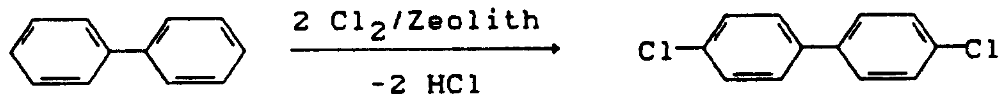


Eine hohe Selektivität der 4,4'-Dihalogenierung war nach dem Stand der Technik nicht zu erwarten. Zwar ist bereits die para-Monochlorierung von Benzolen, die ausschließlich mit Chlor, Niederalkyl oder niederem Alkoxy substituiert sind, in Gegenwart von Faujasit bzw. Zeolith L beschrieben worden (EP 112 722, EP 118 851, Stud. Surf. Sci. Catal. (Amsterdam) 28 (1986) 747-754), wobei etwas höhere Selektivitäten für die para-Chlorierung angegeben worden waren. Eine Bishalogenierung von 2-Kern-Aromaten in Gegenwart von Zeolithen ist jedoch bisher nicht beschrieben worden, weil offenbar ein Vorurteil überwunden werden mußte. Die genannte Monochlorierung an Benzolen mit Zeolithen verschiebt nämlich das ortho-para-Verhältnis von 1,1 bei der konventionellen Chlorierung nur bis zu 0,68 bei der Chlorierung mit Zeolithen, wie durch eine Nacharbeitung gefunden wurde. Diese Ergebnisse mußten im Hinblick auf die vergrößerte Anzahl von möglichen Substitutionsmustern beim Biphenyl als völlig unzureichend erscheinen und sind jedenfalls in keiner Weise geeignet, die starke Verbesserung der 4,4'-Selektivität von 8 % (konventionelle Chlorierung) auf bis zu über 80 % (erfindungsgemäße Chlorierung) zu erklären. Diese Leistung des erfindungsgemäßen Verfahrens ist daher völlig überraschend.

Beispielsweise seien neben dem nicht substituierten Biphenyl das Methyl-, Dimethyl-, Ethyl-, Isopropyl-, Hydroxy- und Methoxy-biphenyl als Einsatzmaterial für das erfindungsgemäße Verfahren genannt. Für die Herstellung asymmetrischer 4,4'-Dihalogen-biphenyle, wie beispielsweise 4-Brom-4'-chlorbiphenyl, 4-Chlor-4'-fluorbiphenyl, 4-Chlor-4'-jodbiphenyl, kommen 4-Chlor-, 4-Fluor-, 4-Brom- oder 4-Jod-biphenyl als Einsatzmaterial in Frage.

Als Halogenierungsmittel eignen sich $Cl_2$, $Br_2$, $SO_2Cl_2$ und $SO_2Br_2$. In bevorzugter Weise werden $Cl_2$ bzw. $Br_2$ eingesetzt. Das Halogenierungsmittel wird in der Regel im stöchiometrischen Verhältnis zum Biphenyl eingesetzt, also bei noch nicht halogenierten Biphenylen im Molverhältnis 2:1, bei bereits monohalogenierten Biphenylen im Molverhältnis 1:1. Von diesem stöchiometrischen Verhältnis kann bis zu 35 Mol-%, bevorzugt bis zu 20 Mol-%, nach oben oder nach unten abgewichen werden.

Das erfindungsgemäße Verfahren wird in Gegenwart von Zeolithen als Katalysatoren durchgeführt. Zeolithe sind kristalline Alumosilikate, die aus einem Netzwerk von $SiO_4$- bzw. $AlO_4$-Tetraedern aufgebaut sind. Die einzelnen Tetraeder sind mit Sauerstoffbrücken über die Ecken untereinander verknüpft und bilden ein räumliches Netzwerk, das von Kanälen und Hohlräumen durchzogen ist. Als Ausgleich für die negative Ladung des Gitters sind austauschbare Kationen eingelagert.

Al in den Zeolithen kann zumindest teilweise durch andere Elemente ersetzt sein, so daß die obengenannte Formel (III) zu ihrer Darstellung in Frage kommt.

Eine ausführliche Darstellung von Zeolithen ist beispielsweise in der Monographie von D. W. Breck "Zeolite Molecular Sieves, Structure, Chemistry, and Use", J. Wiley and Sons, New York, 1974 gegeben. Zeolithe, die für das erfindungsgemäße Verfahren geeignet sind, weisen Porenweiten von mindestens 5 Å, beispielsweise im Bereich von 5-9 Å, bevorzugt 5-7 Å, auf und haben ein Verhältnis n/m = 1-3000, bevorzugt 1-2000. In der Formel (III) bedeutet $Me^1$ ein dreiwertiges Element aus der Gruppe Al, Ga, In, B und V.

In bevorzugter Weise ist $Me^1$ Al allein

In Frage kommen besonders Zeolithe des Strukturtyps Faujasit, L, Offretit, Gmelinit, Cancrinit, H, ZSM 12, ZSM 25, Zeolith $\beta$, Ferrierit, ZSM 5, ZSM 11, Heulandit, ZSM 22, ZSM 23, ZSM 48, ZSM 43, ZSM 35, PSH-3, Zeolith $\rho$, ZSM 38, CSZ-1, ZSM 3, ZSM 20, Mordenit, Zeolith $\Omega$, Erionit, Borsilikat, besonders bevorzugt Zeolithe des Strukturtyps Mordenit, Ferrierit, H, L, $\Omega$, ZSM 11 oder ZSM 5, ganz besonders bevorzugt vom Strukturtyp $\Omega$ oder L.

Die genannten Zeolithtypen können mit austauschbaren Kationen aus ihren Synthese formen oder irgendeiner Vielzahl anderer Kationen im Sinne eines Ionenaustauschs versehen seien. Dieser Austausch ist Stand der Technik und dem Fachmann wohl bekannt. Erfindungsgemäß geeignet sind sowohl Zeolithe in ihrer H-Form als auch Zeolithe, in denen der Wasserstoff ganz oder teilweise gegen Alkalimetallionen, bevorzugt $Na^+$ oder $K^+$ ausgetauscht sind. Vielfach eignen sich auch Formen, bei denen der Wasserstoff teilweise oder ganz gegen zwei verschiedene Metallkationen, beispielsweise gegen die Kombination Na/K ausgetauscht ist. Der besonders bevorzugte Strukturtyp L kann beispielsweise als Kationen solche von H, Na, K oder Gemische von ihnen, bevorzugt von Na, K oder Gemische von ihnen, besonders bevorzugt von

K enthalten.

Der Zeolith-Katalysator wird in einer Menge von 1-100 Gew.-%, bevorzugt 5-50 Gew.-%, besonders bevorzugt 10-30 Gew.-%, bezogen auf das Gewicht des umzusetzenden Biphenyls, eingesetzt.

Es wurde weiterhin gefunden, daß die Selektivität der Zeolith-Katalysatoren durch Co-Katalysatoren erhöht werden kann. Co-Katalysatoren sind polare Substanzen, die vermutlich mit aktiven Zentren der Zeolith-Katalysatoren in Wechselwirkung treten können. Co-Katalysatoren gehören beispielsweise der folgenden Gruppe an: Wasser, Alkohole, Aldehyde oder deren Acetale, Ketone, Carbonsäuren oder deren Salze, deren Halogenide, deren Amide oder deren Ester, Nitrile, Schwefel, Schwefelhalogenide, Mercaptane, Thioether, Thiocarbonsäuren, Amine oder deren Salze, quartäre Ammoniumsalze, Jod. Als Einzelverbindungen seien beispielsweise genannt: Wasser, Methanol, Ethanol, (Iso)propanol, Butanol Chlorethanol, Formaldehyd, Acetaldehyd, Butyraldehyd, Benzaldehyd, Acetaldehyd-dimethylacetal, Aceton, Methylethylketon, Benzophenon, Essigsäure, Chloressigsäure, Dichloressigsäure, Methoxyessigsäure, Propionsäure, Capronsäure, Essig-bzw. Propionsäurechlorid, Acet-dimethylamid, Chloracetamid, Essigsäureethylester, Buttersäuremethylester, Acetonitril, Propionitril, Capronitril, Benzonitril, Kaliumformiat, Natriumacetat, Kalium-chloracetat, Calciumpropionat, Zinnbutyrat, Schwefeldichlorid, Butylmercaptan, Thiophenol, Dimethylsulfid, Dibutylsulfid, Dibutyldisulfid, Diphenylsulfid, Thioessigsäure, Dimethylamin, Ethylamin, Butylamin, Pyridin, Picolin, Chinolin, die Salze der genannten Amine, Tetramethylammoniumchlorid, Tetraethylammoniumchlorid, $J_2$.

Solche Co-Katalysatoren werden in einer Menge von 0,01 bis 50 Gew.-%, bevorzugt 0,02-20 Gew.-%, bezogen auf das Gewicht des eingesetzten Zeolith-Katalysators, zugesetzt Die Menge des Co-Katalysators hängt teilweise von der Polarität der gewählten Substanz ab, ist im allgemeinen sehr variabel, wird aber mit Vorteil im unteren Bereich, beispielswiese in Mengen von 0,02-2 Gew.-% verwendet.

Das erfindungsgemäße Verfahren kann mit oder ohne zusätzliches Lösungsmittel durchgeführt werden. Solche Lösungsmittel müssen gegenüber den verwendeten Halogenierungsmitteln resistent sein; in Frage kommen beispielsweise: Kohlenwasserstoffe oder Halogenkohlenwasserstoffe, wie Petrolether, Methylenchlorid, Chloroform, Tetrachlorkohlenstoff, 1,1,1-Trichlorethan, 1,2-Dichlorethan, Perchlorethan, Perchlorethylen, niedere Carbonsäuren, wie Essigsäure und andere, die dem Fachmann als inert bekannt sind. In bevorzugter Weise wird jedoch in der Schmelze des umzusetzenden Biphenyls gearbeitet, wodurch die Aufarbeitung des Reaktionsansatzes vereinfacht wird. Das erfindungsgemäße Verfahren kann weiterhin in der Gasphase durchgeführt werden. Die Durchführung in der Flüssigphase ist jedoch bevorzugt.

Weiterhin kann kontinuierlich oder diskontinuierlich, bei Normaldruck, vermindertem oder erhöhtem Druck gearbeitet werden; der Druck ist für den Ablauf des erfindungsgemäßen Verfahrens nicht kritisch und hat beispielsweise nur Bedeutung, wenn bei erhöhter Temperatur mit einem leicht flüchtigen Lösungsmittel gearbeitet werden soll. Hierbei wird vorteilhaft der Eigendruck des Systems angewandt.

Das erfindungsgemäße Verfahren läßt sich in einem weiten Temperaturbereich durchführen, beispielsweise bei 0 bis 300 ° C, bevorzugt 30 bis 180 ° C, besonders bevorzugt bei 50 bis 150 ° C.

Das erfindungsgemäße Verfahren wird beispielsweise so durchgeführt, daß einer diskontinuierlichen Arbeitsweise Biphenyl unter Rühren aufgeschmolzen oder in einem der genannten Lösungsmittel gelöst wird. Sodann wird der Zeolith-Katalysator in gepulverter Form zugefügt und danach 2 Mol des Halogenierungsmittels in dem Maße, wie es verbrauch wird, bei Reaktionstemperatur in die flüssige Phase eingeleitet. Für die kontinuierliche Durchführung eignen sich beispielsweise Säulenapparaturen, in denen der Zeolith-Katalysator in stückiger Form angeordnet wird. Der eingesetzte Co-Katalysator befindet sich im allgemeinen in der flüssigen Phase der Schmelze oder Lösung. Für Isolierung der Reinigung der erfindungsgemäß erhältlichen 4,4′-Dihalogeno-biphenyle werden allgemein bekannte Verfahren, wie Destillation, Vakuumdestillation, Umkristallisation oder chromatographische Verfahren eingesetzt. Vielfach zeigt das gewünschte 4,4′-Isomere einen deutlich höheren Schmelzpunkt und eine deutlich geringere Löslichkeit, verglichen mit etwa mitentstehenden halogenierten Biphenylen anderer Substitutionsmuster. Dreifach oder noch höher halogenierte Biphenyle treten nur in untergeordneten Mengen, beispielsweise in einer Menge von höchstens 2 Gew.-%, vielfach jedoch in noch geringerer Menge, auf. Dies ist wegen der Giftigkeit solcher höher halogenierter Biphenyle von großem Vorteil bezüglich der Arbeitssicherheit und Umweltbelastung und fördert weiterhin die Wirtschaftlichkeit des erfindungsgemäßen Verfahrens. In 4- oder 4′-Stellung monosubstituierte Biphenyle können wieder in das erfindungsgemäße Verfahren zurückgeführt werden, wobei sich ebenfalls die Wirtschaftlichkeit steigern läßt.

Der entweder als Destillationsrückstand oder als Extraktionsrückstand hinterbleibende Zeolith-Katalysator kann im allgemeinen ohne weitere Aktivierung erneut erfindungsgemäß eingesetzt werden. Für den Fall, daß nach mehrmaliger Wiederverwendung eine Aktivitätsminderung beobachtet wird, kann der Zeolith-Katalysator nach einem üblichen Verfahren, beispielsweise durch Calcinierung bei erhöhter Temperatur (beispielsweise 400-600 ° C) reaktiviert werden.

Beispiele

In den Beispielen 1 bis 15 und 24-28 wurden folgende Zeolithe eingesetzt:

| Beispiel | Typ/Kation | $SiO_2/Al_2O_3$ |
|---|---|---|
| 1 | K-Zeolith L | 6 |
| 2 | Na, K-Zeolith L | 6,4 |
| 3 | H-Zeolith L | 7,5 |
| 4 | H-Mordenit | 25 |
| 5 | H-Mordenit | 16 |
| 6 | H-Mordenit | 18 |
| 7 | K-Zeolith Ω | 6,2 |
| 8 | H-Ferrierit | 17 |
| 9 | H-ZSM 11 | 65 |
| 10 | H-Offretit/H-Erionit | 6 |
| 11 | Borsilikat | $SiO_2/B_2O_3$ = 40 |
| 12 | Na-Zeolith X | 2,5 |
| 13 | K-Zeolith H | 4 |
| 14 | Na/K-Zeolith L | 6 |
| 15 | K-Zeolith Ω | 6,2 |
| 24 | Na, K-Zeolith L | 6,4 |
| 25 | K-Zeolith L | 6 |
| 26 | K-Zeolith Ω | 6,2 |
| 27 | H-ZSM 5 | 90 |
| 28 | H-ZSM 11 | 65 |

In den Vergleichsbeispielen I bis V wurden bei sonst gleicher Verfahrensweise wie in Beispiel 1 an Stelle der Zeolithe folgende Katalysatoren eingesetzt:

| Vergleichsbeispiel | Katalysator |
|---|---|
| I | ohne |
| II | 2 g $FeCl_3$ |
| III | 2 g $FeCl_3$ + 2 g Thiophen |
| IV | 7 g 4,4′-Dichlor-diphenylthioether |
| V | 2,5 g $FeCl_3$ + 2,5 g 4,4′-Dichlor-diphenylthioether |

Beispiele 1 bis 13

Man brachte 77,1 g (0,5 Mol) Biphenyl in einer Rührapparatur unter Rühren und Durchleiten von Stickstoff zum Schmelzen, fügte 15 g aktives (3 Std. bei 400 °C aktiviertes) Zeolithpulver zu und leitete bei 100 °C innerhalb von 5 h 71 g (1 Mol) $Cl_2$-Gas ein. Man hielt noch 30 Minuten bei 100 °C, entgaste die Schmelze mit Stickstoff und bestimmte deren Zusammensetzung gaschromatographisch.

Die Ergebnisse werden zusammen mit denen der Beispiele 14 und 15 sowie der Vergleichsbeispiele I bis V in der Tabelle 1 dargestellt.

Beispiel 14

77,1 g (0,5 Mol) Biphenyl wurden unter Zusatz von 15 g K/Na-L-Zeolith unter Stickstoff aufgeschmolzen, dan bei 100 °C 135 g (1 Mol) Sulfurylchlorid unter Rühren in die Schmelze innerhalb von 3 h eingetropft. Nach Beendigung der Gasentwicklung spülte man mit Stickstoff und bestimmte die Zusammensetzung der Schmelze gaschromatographisch.

Beispiel 15

Man verfuhr analog Beispiel 14, wobei anstelle des K/Na-Zeoliths L 15 g K-Ω-Zeolith eingesetzt wurden.

Tabelle 1: Produktzusammensetzung in %

| | Monochlor-biphenyl | | Dichlorbiphenyle | | | | | | | Trichlorbiphenyle | | | |
|---|---|---|---|---|---|---|---|---|---|---|---|---|---|
| Vergleichs-beispiel | 2- | 4- | 2,2'- | * | 2,3'- | 2,4'- | 3,3'- | 3,4'- | 4,4'- | * | * | * | Rest (z.T. unbekannt) |
| I | 14,5 | 38,6 | - | 3,57 | - | 0,50 | - | 2,17 | 1,55 | - | - | - | 38,5 Biphenyl |
| II | 18,9 | 7,89 | 11,7 | 4,66 | 5,73 | 20,97 | - | 2,24 | 8,04 | 1,77 | 1,99 | 0,87 | 15,3 |
| III | 22,8 | 10,51 | 11,1 | 4,87 | 5,48 | 21,34 | - | 2,41 | 8,61 | 1,11 | 1,46 | 0,62 | 9,7 |
| IV | 33,90 | 25,96 | 1,26 | 2,71 | 0,48 | 7,45 | - | 1,77 | 5,51 | 0,30 | 0,19 | 0,45 | 7,5 Biphenyl + 12,5 unbekannt |
| V | 28,43 | 3,53 | 9,53 | 3,18 | 2,95 | 27,82 | - | 0,83 | 16,38 | 1,59 | 0,66 | 0,50 | 0,6 |
| Beispiel | | | | | | | | | | | | | |
| 1 | 6,43 | 3,46 | 2,07 | 1,27 | 1,57 | 16,20 | - | 1,36 | 64,14 | 0,48 | 0,99 | 1,03 | 1,0 |
| 2 | 15,02 | 10,84 | 0,98 | 0,96 | 0,77 | 11,76 | 0,05 | 1,50 | 56,13 | 0,13 | 0,50 | 0,36 | 1,0 |
| 3 | 9,62 | 3,75 | 6,18 | 1,56 | 3,25 | 24,0 | 0,36 | 1,60 | 43,32 | 0,43 | 0,85 | 0,83 | 4,24 |
| 4 | 19,15 | 9,53 | 1,54 | 2,42 | 0,85 | 22,20 | 0,17 | 1,70 | 37,88 | 2,40 | - | 0,76 | 1,42 |
| 5 | 16,70 | 3,90 | 2,27 | 1,80 | 1,13 | 25,76 | 0,01 | 1,25 | 42,05 | 2,18 | 0,75 | - | 2,22 |
| 6 | 18,64 | 3,88 | 1,16 | 1,72 | 0,73 | 22,00 | - | 1,50 | 45,90 | 2,34 | - | 0,97 | 1,16 |
| 7 | 20,48 | 1,45 | 2,96 | 1,59 | 1,36 | 23,92 | 0,09 | 1,55 | 44,19 | - | 1,04 | 0,79 | 0,57 |
| 8 | 19,42 | 0,60 | 5,35 | 1,60 | 1,57 | 32,52 | - | 0,84 | 35,25 | 0,90 | 0,57 | - | 1,37 |
| 9 | 21,69 | 0,92 | 3,82 | 2,09 | 2,13 | 29,60 | 0,05 | 2,01 | 33,36 | 1,58 | - | 1,01 | 1,72 |
| 10 | 13,04 | 0,30 | 5,51 | 1,48 | 1,89 | 37,37 | 0,33 | 0,70 | 31,72 | 1,15 | 2,82 | 1,78 | 1,92 |
| 11 | 23,18 | 3,14 | 4,54 | 2,28 | 2,17 | 27,91 | - | 2,00 | 31,42 | 0,53 | 1,32 | 0,80 | 0,80 |
| 12 | 14,89 | 0,68 | 7,80 | 2,24 | 2,99 | 32,98 | 0,03 | 1,28 | 32,14 | 1,45 | 0,45 | 0,95 | 1,1 |
| 13 | 22,10 | 3,16 | 3,13 | 1,26 | 1,23 | 26,30 | - | 1,28 | 39,67 | 0,6 | 0,15 | 0,54 | 0,58 |
| 14 | 11,03 | 6,94 | 0,49 | 0,39 | 0,39 | 10,93 | - | 1,04 | 67,05 | - | 0,15 | 0,14 | 1,45 |
| 15 | 21,34 | 1,46 | 3,04 | 1,14 | 1,28 | 24,23 | - | 1,41 | 42,87 | - | 0,53 | 0,62 | 2,08 |

* unbekannter Substitutionsort

Beispiele 16 bis 20

Bei gleicher Verfahrensweise wie in Beispiel 1 wurden zusammen mit 15 g des Katalysators Zeolith K-L folgende Zusätze mitverwendet.

| | |
|---|---|
| Beispiel 16 | 2 g Chloressigsäure |
| Beispiel 17 | 2 g Chloressigsäurechlorid |
| Beispiel 18 | 1 g Wasser |
| Beispiel 19 | 2 g Chloressigsäure-Natriumsalz |
| Beispiel 20 | 2 g Kaliumacetat |

Die Ergebnisse sind in Tabelle 2 zusammengestellt.

Beispiele 21 bis 23

Man verfuhr analog Beispiel 1 unter Verwendung von 15 g K/Na-Zeolithe L(nach Beispiel 2), jedoch bei unterschiedlichen Reaktionstemperaturen:

| | |
|---|---|
| Beispiel 21 | 70 ° C |
| Beispiel 2 | 100 ° C |
| Beispiel 22 | 120 ° C |
| Beispiel 23 | 150 ° C |

Die Ergebnisse gehen aus Tabelle 3 hervor.

Tabelle 2: Produktzusammensetzung in % (Angaben analog Tabelle 1)

| Beispiel | Monochlor-biphenyl | | Dichlor-biphenyle | | | | | | | Rest |
|---|---|---|---|---|---|---|---|---|---|---|
| | 2- | 4- | 2,2' | * | 2,3' | 2,4' | 3,3' | 3,4' | 4,4' | |
| 16 | 2,76 | 3,58 | 0,39 | 0,71 | 0,28 | 7,93 | - | 0,78 | 81,76 | 1,81 |
| 17 | 9,96 | 3,79 | 1,30 | 1,46 | 0,96 | 13,15 | - | 1,69 | 66,33 | 1,63 |
| 18 | 10,23 | 1,22 | 2,65 | 1,28 | 1,71 | 16,79 | - | 1,76 | 62,03 | 2,33 |
| 19 | 4,20 | 10,97 | 0,58 | 0,86 | 0,40 | 9,88 | - | 1,12 | 70,39 | 1,60 |
| 20 | 3,80 | 1,36 | 1,39 | 0,97 | 0,93 | 13,54 | - | 1,24 | 74,76 | 2,01 |

* unbekannter Substitutionsort

<u>Tabelle 3</u> Temperaturabhängigkeit der Biphenylchloriung; Produktzusammensetzung in %
(Angaben analog Tabelle 1)

| Beispiel/ Temperatur | | Monochlor-biphenyl 2- | 4- | Dichlor-biphenyle 2,2' | * | 2,3' | 2,4' | 3,4' | 4,4' | Trichlorbiphenyle * | * | * | Rest |
|---|---|---|---|---|---|---|---|---|---|---|---|---|---|
| 21 | 70° C | 9,303 | 9,523 | 1,090 | 0,831 | 0,714 | 13,853 | 1,054 | 59,699 | 0,479 | 0,822 | 0,592 | 2,040 |
| 2 | 100° C | 15,023 | 10,840 | 0,981 | 0,958 | 0,770 | 11,760 | 1,503 | 56,130 | 0,132 | 0,500 | 0,355 | 1,048 |
| 22 | 120° C | 8,559 | 0,662 | 3,094 | 1,315 | 2,106 | 19,479 | 1,807 | 59,371 | 0,484 | 1,066 | 1,048 | 1,009 |
| 23 | 150° C | 5,305 | 0,409 | 4,414 | 1,533 | 3,010 | 23,186 | 1,760 | 53,713 | 0,991 | 1,633 | 1,803 | 2,243 |

* unbekannter Substitutionsort

## Beispiel 24

77,1 g (0,5 Mol) Biphenyl wurden analog Beispiel 1 in Gegenwart von 15 g Zeolith Na/K-L aufgeschmolzen; dann wurden 159,8 g (1 Mol) $Br_2$ im Verlauf von 8 h bei 100 °C in die Schmelze eingetropft. Man

rührte noch 30 Minuten nach, spülte 30 Minuten durch Hindurchleiten von Stickstoff und bestimmte die Zusammensetzung gaschromatographisch: der Umsatz betrug 100 %, die Selektivität für 4,4′-Dibrombiphenyl 75 %, die für 4-Monobrombiphenyl 16,5 %.

Beispiele 25 bis 28

Bei gleicher Verfahrensweise wie in Beispiel 24 wurden weitere Zeolithe eingesetzt. Der Umsatz, bezogen auf das eingesetzte Biphenyl, betrug jeweils 100 %. Die verwendeten Zeolithe sowie die erreichten Selektivitäten gehen aus folgender Aufstellung hervor:

| Beispiel | Katalysator | [S] 4,4′-Dibrom- | [S] 4-Monobrombiphenyl |
|---|---|---|---|
| 25 | K-L | 78,9 | 11,0 |
| 26 | K-Ω | 81,5 | 8,8 |
| 27 | H-ZSM 5 | 66,8 | 16,5 |
| 28 | H-ZSM 11 | 69,5 | 13,9 |

## Patentansprüche

**1.** Verfahren zur Herstellung von 4,4'-Dihalogeno-biphenylen der Formel

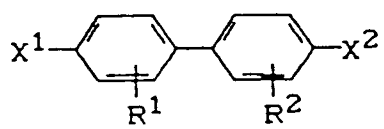

durch katalysierte Halogenierung von Biphenylen der Formel

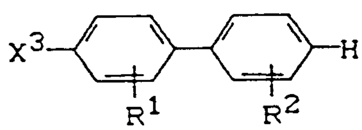
,

wobei in den Formeln

- $X^1$ und $X^2$ unabhängig voneinander für Chlor oder Brom, bevorzugt für Chlor stehen,
- $X^3$ für Wasserstoff, Chlor oder Brom, bevorzugt für Wasserstoff oder Chlor, besonders bevorzugt für Wasserstoff steht und
- $R^1$ und $R^2$ unabhängig voneinander Wasserstoff, $C_1$-$C_4$-Alkyl (bevorzugt $C_1$-$C_2$-Alkyl, besonders bevorzugt Methyl), $C_1$-$C_4$-Alkoxy (bevorzugt $C_1$-$C_2$-Alkoxy, besonders bevorzugt Methoxy), Hydroxy, Fluor, Chlor oder Brom bedeuten,

dadurch gekennzeichnet, daß man die Biphenyle mit Halogenierungsmitteln aus der Gruppe von $Cl_2$, $Br_2$, $SO_2Cl_2$ und $SO_2Br_2$ in Gegenwart von Zeolithen der Formel

$$M_m [mMe^1O_2 \cdot nSiO_2] \cdot q\ H_2O\ ,$$

worin

- M ein austauschbares Kation aus der Gruppe von Wasserstoff und den Alkalimetallen bedeutet,
- $Me^1$ ein dreiwertiges Element aus der Gruppe von Al, Ga, In, B und V darstellt,
- n/m das Verhältnis der Elemente bedeutet und Werte von 1 - 3000 annimmt und
- q die Menge des sorbierten Wassers bedeutet,

wobei die Zeolithe Porenweiten von mindestens 5 Å haben, umsetzt.

**2.** Verfahren nach Anspruch 1, dadurch gekennzeichnet, daß $Me^1$ Aluminium ist.

**3.** Verfahren nach Anspruch 1, dadurch gekennzeichnet, daß Zeolithe des Strukturtyps Faujasit, L, Offretit, Gmelinit, Cancrinit, H, ZSM 12, ZSM 25, Zeolith ß, Ferrierit, ZSM 5, ZSM 11, Heulandit, ZSM 22, ZSM 23, ZSM 48, ZSM 43, ZSM 35, PSH-3, Zeolith ρ, ZSM 38, CSZ-1, ZSM 3, ZSM 20, Mordenit, Zeolith Ω, Erionit, Borsilikat, bevorzugt Zeolithe des Strukturtyps, Mordenit, Ferrierit, H, L, Ω, ZSM 5 oder ZSM 11, besonders bevorzugt vom Strukturtyp Ω oder L, eingesetzt werden.

**4.** Verfahren nach Anspruch 3, dadurch gekennzeichnet, daß Zeolith des Strukturtyps Ω oder L eingesetzt werden, bei denen die austauschbaren Kationen Ionen von Wasserstoff, Natrium, Kalium oder Gemische von ihnen, bevorzugt von Natrium, Kalium oder Gemische von ihnen, besonders bevorzugt von Kalium sind.

**5.** Verfahren nach Anspruch 1, dadurch gekennzeichnet, daß in Gegenwart von 1-100 Gew.-%, bevorzugt 5-50 Gew.-%, besonders bevorzugt 10-30 Gew.-% Zeolith, bezogen auf das Gewicht des umzusetzenden Biphenyls, gearbeitet wird.

**6.** Verfahren nach Anspruch 1, dadurch gekennzeichnet, daß in Gegenwart von Co-Katalysatoren aus der Gruppe: Wasser, Alkohole, Aldehyde oder deren Acetale, Ketone, Carbonsäuren oder deren Salze, deren Halogenide, deren Amide oder deren Ester, Nitrile, Schwefel, Schwefelhalogenide, Mercaptane, Thioether, Thiocarbonsäuren, Amine oder deren Salze, quartäre Ammoniumsalze oder Iod gearbeitet wird.

**7.** Verfahren nach Anspruch 6, dadurch gekennzeichnet, daß in Gegenwart von 0,01-50 Gew.-%, bevorzugt 0,02 bis 20 Gew.-% an Co-Katalysator, bezogen auf das Gewicht des eingesetzten Zeolith-Katalysators, gearbeitet wird.

**8.** Verfahren nach Anspruch 1, dadurch gekennzeichnet, daß in der Schmelze des Biphenyls ohne weiteres Lösungsmittel gearbeitet wird.

**9.** Verfahren nach Anspruch 1, dadurch gekennzeichnet, daß unsubstituiertes Biphenyl oder sein 4-Monohalogenderivat eingesetzt wird.

**Claims**

**1.** Process for the preparation of 4,4'-dihalogenobiphenyls of the formula

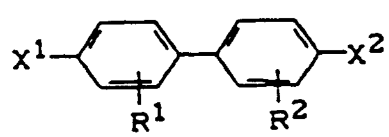

by catalysed halogenation of biphenyls of the formula

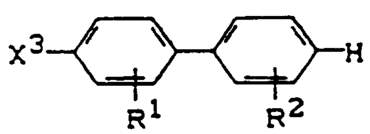

,

in which the formulae
$X^1$ and $X^2$ independently of one another represent chlorine or bromine, preferably chlorine,
$X^3$ represents hydrogen, chlorine or bromine, preferably hydrogen or chlorine, particularly preferably hydrogen and
$R^1$ and $R^2$ independently of one another denote hydrogen, $C_1$-$C_4$-alkyl (preferably $C_1$-$C_2$-alkyl, particularly preferably methyl), $C_1$-$C_4$-alkoxy (preferably $C_1$-$C_2$-alkoxy, particularly preferably methoxy), hydroxyl, fluorine, chlorine or bromine,
characterised in that the biphenyls are reacted with halogenating agents from the group comprising $Cl_2$, $Br_2$, $SO_2Cl_2$ and $SO_2Br_2$ in the presence of zeolites of the formula

$M_m [nMe'O_2 \cdot nSiO_2] \cdot q\ H_2O$

in which
M denotes an exchangeable cation from the group comprising hydrogen and the alkali metals,
$Me^1$ represents a trivalent element from the group comprising Al, Ga, In, B and V,
n/m denotes the ratio of the elements and adopts values of 1 - 3,000 and
q denotes the amount of the water absorbed,
the zeolites having pore sizes of at least 5Å.

**2.** Process according to Claim 1, characterised in that $Me^1$ is aluminium.

**3.** Process according to Claim 1, characterised in that the zeolites used are those of the structure type faujasite, L, offretite, gmelinite, cancrinite, H, ZSM 12, ZSM 25, zeolite $\beta$, ferrierite, ZSM 5, ZSM 11, heulandite, ZSM 22, ZSM 23, ZSM 48, ZSM 43, ZSM 35, PSH-3, zeolite $\rho$, ZSM 38, CSZ-1, ZSM 3, ZSM 20, mordenite, zeolite Ω, erionite, boron silicate, preferably zeolites of the structure type mordenite, ferrierite, H, L, Ω, ZSM 5 or ZSM 11, particularly preferably of the structure type Ω or L.

**4.** Process according to Claim 3, characterised in that zeolites of the structure type Ω or L are used in which the exchangeable cations are ions of hydrogen, sodium, potassium or mixtures thereof, preferably of sodium, potassium or mixtures thereof, particularly preferably of potassium.

**5.** Process according to Claim 1, characterised in that the reaction is carried out in the presence of 1-100% by weight, preferably 5-50% by weight, particularly preferably 10-30% by weight, of zeolite, relative to the weight of the biphenyl to be reacted.

**6.** Process according to Claim 1, characterised in that the reaction is carried out in the presence of co-catalysts from the group: water, alcohols, aldehydes or acetals thereof, ketones, carboxylic acids or salts thereof, halides thereof, amides thereof or esters thereof, nitriles, sulphur, sulphur halides, mercaptans, thioethers, thiocarboxylic acids, amines or salts thereof, quarternary ammonium salts or iodine.

**7.** Process according to Claim 6, characterised in that the reaction is carried out in the presence of 0.01-50% by weight, preferably 0.02 to 20% by weight of co-catalyst, relative to the weight of the zeolite catalyst used.

**8.** Process according to Claim 1, characterised in that the reaction is carried out in the melt of the biphenyl without a further solvent.

**9.** Process according to Claim 1, characterised in that unsubstituted biphenyl or its 4-monohalogeno derivative is used.

**Revendications**

**1.** Procédé pour la fabrication de 4,4'-dihalogéno-biphényles de formule

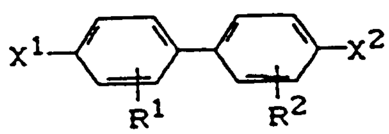

par halogénation catalytique de biphényles de formule

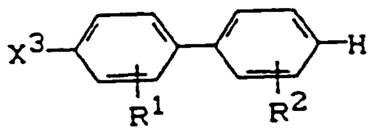

dans lesquelles

$X^1$ et $X^2$ représentent indépendamment l'un de l'autre le chlore ou le brome, de préférence le chlore,

$X^3$ représente l'hydrogène, le chlore ou le brome, de préférence l'hydrogène ou le chlore, de préférence particulière l'hydrogène, et

$R^1$ et $R^2$ représentent indépendamment l'un de l'autre l'hydrogène, alkyle en $C_1$-$C_4$ (de préférence alkyle en $C_1$-$C_2$, de préférence particulière méthyle), alcoxy en $C_1$-$C_4$ (de préférence alcoxy en $C_1$-$C_2$, en particulier méthoxy), hydroxy, le fluor, le chlore ou le brome,

caractérisé en ce que l'on fait réagir les biphényles avec des agents d'halogénation du groupe comportant $Cl_2$, $Br_2$, $SO_2Cl_2$ et $SO_2Br_2$ en présence de zéolites de formule

$$M_m [mMe^1O_2 \cdot nSiO_2] \cdot q\ H_2O$$

dans laquelle

M représente un cation échangeable choisi dans le groupe comportant l'hydrogène et les métaux alcalins,

$Me^1$ représente un élément trivalent choisi dans le groupe comportant Al, Ga, In, B et V,

n/m représente le rapport entre les éléments et peut avoir une valeur de 1 à 3.000, et

q représente la quantité de l'eau sorbée,

la zéolite ayant une dimension de pores d'au moins 5 Å.

2. Procédé selon la revendication 1, caractérisé en ce que $Me^1$ est l'aluminium.

3. Procédé selon la revendication 1, caractérisé en ce que l'on utilise des zéolites de structure de type faujasite, L, offrétite, gmélinite, cancrinite, H, ZSM 12, ZSM 25, zéolite $\beta$, ferreriérite, ZSM 5, ZSM 11, heulandite, ZSM 22, ZSM 23, ZSM 48, ZSM 43, ZSM 35, PSH-3, zéolite $\rho$ , ZSM 38, CSZ-1, ZSM 3, ZSM 20, mordénite, zéolite Ω, érionite, borosilicate, de préférence des zéolites de structure de type mordénite, ferriérite, H, L, Ω, ZSM 5 ou ZSM 11, de préférence particulière des zéolites de structure de type Ω ou L.

4. Procédé selon la revendication 3, caractérisé en ce que l'on utilise des zéolites de structure de type Ω ou L, dans lesquelles les cations échangeables sont des ions hydrogène, sodium, potassium ou des mélanges de ces ions, de préférence des ions sodium, potassium ou leurs mélanges, de préférence particulière des ions potassium.

5. Procédé selon la revendication 1, caractérisé en ce qu'on travaille en présence de 1 à 100% en poids, de préférence 5 à 50% en poids, de préférence particulière 10 à 30% en poids de zéolite, par rapport au poids du biphényle à faire réagir.

6. Procédé selon la revendication 1, caractérisé en ce qu'on travaille en présence de co-catalyseurs du groupe comportant l'eau, des alcools, des aldéhydes ou leurs acétals, des cétones, des acides carboxyliques ou leurs sels, leurs halogénures, leurs amides ou leurs esters, des nitriles, le soufre, les halogénures de soufre, les mercaptans, les thioéthers, les acides thiocarboxyliques, des amines ou leurs sels, les sels d'ammonium quaternaires, ou l'iode.

7. Procédé selon la revendication 6, caractérisé en ce qu'on travaille en présence de 0,01 à 50% en poids, de préférence 0,02 à 20% en poids de cocatalyseur par rapport au poids du catalyseur zéolite utilisé.

8. Procédé selon la revendication 1, caractérisé en ce qu'on travaille dans la masse fondue de biphényle sans addition de solvant.

9. Procédé selon la revendication 1, caractérisé en ce qu'on emploie du biphényle substitué ou son dérivé monohalogéné en 4.